# EUROPEAN PATENT SPECIFICATION

(11) **EP 2 503 906 B1**
(45) Date of publication and mention of the grant of the patent: **30.03.2016**
(21) Application number: 10782160.5
(22) Date of filing: 19.11.2010
(51) Int. Cl.: A23L 33/00, A23L 33/10, A23L 33/17

(54) **NUTRITIONAL COMPOSITIONS INCLUDING A HIGH PROTEIN COMPONENT AND EXOGENOUS NUCLEOTIDES**
ERNÄHRUNGSZUSAMMENSETZUNGEN MIT EINEM BESTANDTEIL MIT HOHEM PROTEINGEHALT UND EXOGENEN NUKLEOTIDEN
COMPOSITIONS NUTRITIONNELLES COMPRENANT UN CONSTITUANT À HAUTE TENEUR EN PROTÉINES ET DES NUCLÉOTIDES EXOGÈNES

(30) Priority: 25.11.2009 US 264405 P; 13.10.2010 US 392699 P
(43) Date of publication of application: 03.10.2012
(73) Proprietor: Nestec S.A., 1800 Vevey (CH)
(72) Inventor: GREENBERG, Norman Alan, New Hope, Minnesota 55427 (US); MILLER, Kevin Burke, Minneapolis, Minnesota 55404 (US); ROUGHEAD, Zamzam (Fariba) Kabiry, Plymouth, Minnesota 55446 (US)
(74) Representative: Stephen, Paula-Marie
(86) International application number: PCT/US2010/057310
(87) International publication number: WO 2011/066175

(56) References cited:
- EP-A1- 1 800 675
- WO-A1-01/52663
- WO-A1-96/02137
- WO-A1-2004/103383
- WO-A1-2009/038458
- US-A- 5 700 590
- US-A1- 2002 106 436
- US-A1- 2005 272 686
- US-A1- 2007 104 761
- US-B1- 6 342 484

## Description

### BACKGROUND

The present disclosure generally relates to health and nutrition. More specifically, the present disclosure relates to nutritional compositions including protein and an exogenous nucleotide and methods of making and using the nutritional compositions.

There are many types of nutritional compositions currently on the market. Nutritional compositions can be targeted toward certain consumer types, for example, young, elderly, athletic, etc., based on the specific ingredients of the nutritional composition. Nutritional compositions can also be formulated based on the certain physiological conditions that the nutritional compositions are intended to treat or improve.

Patients are more susceptible to wounds during care in the health system when they are malnourished as compared to free living individuals. These patients typically do not consume adequate amounts of protein. Despite the consumption of protein containing products, the prevention of wounds, such as pressure ulcers, is often not successful. Nutritional products can be used as part of a therapy to help heal and close the wounds.

For example, WO2004/103383 relates to a method for the treatment and/or prevention of chronic wounds, by enterally administering to a patient a composition wherein protein represents between 10 and 40 % of the total caloric content of the composition; carbohydrate represents between 15 and 90 % of the total caloric content of the composition; and wherein the protein provides a certain amount of glycine and/or leucine. Protein with high levels of glycine and proline, which relate to an enzyme alteration, are important. Also, leucine is able to increase anabolism.

In US 6 342 484, compositions and preparations for the promotion of wound healing in an animal include a wound healing promoting concentration of nucleotides.

However, the use of these nutritional products does not ensure a successful rapid healing of the various types of wounds. Furthermore, medical conditions such as diabetes will also interfere with wound healing.

It is estimated that 8 - 12% of all hospital patients will acquire a pressure ulcer in the first two weeks after admission. Even more long term care residents acquire pressure ulcers. Pressure ulcers are divided into four categories with stage I being the mildest and stage IV the most severe. The average time to heal these is 4 and 22 weeks for stage I and IV, respectively. This indicates that the current nutritional options for patients with pressure ulcers are not adequate to support rapid healing.

### SUMMARY

Nutritional compositions having a high protein component and an exogenous nucleotide and methods of making and using the nutritional compositions are provided. In a general embodiment, the present disclosure provides a nutritional composition comprising a high protein component and one or more exogenous nucleotides for use in treating a wound or improving wound healing in a mammal.

The high protein component includes one or more proteins, peptides, and amino acids, and precursors and metabolites of proteins, peptides, and amino acids in an amount that provides at least about 18% of the total calories of the nutritional composition.

The nutritional composition has a total volume of 300 mL or less. The high protein component in this nutritional composition includes one or more proteins, peptides, and amino acids, and precursors and metabolites of proteins, peptides, and amino acids in an amount greater than about 14 grams.

In an embodiment, the high protein component includes at least about 5 grams of protein, at least about 3 grams of a bitter tasting amino acid, and at least about 7 grams of a neutral tasting acid. The bitter tasting amino acid can be arginine, phenylalanine, tyrosine, leucine, isoleucine, valine, methionine, histidine or a combination thereof. The neutral tasting acid can be glutamine, glycine, alanine, threonine, praline, serine or a combination thereof.

In an embodiment, the exogenous nucleotide is in an amount of about 1 gram/1000 calories of the nutritional composition. The exogenous nucleotide can be in a monomeric form such as 5' Adenosine Monophosphate, 5'-Guanosine Monophosphate, 5'-Cytosine Monophosphate, 5'-Uracil Monophosphate, 5'-Inosine Monophosphate, 5'-Thymine Monophosphate or a combination thereof. The exogenous nucleotide can be intact ribonucleic acid or other compounds containing nucleotides..

In an embodiment, the nutritional composition further comprises one or more ingredients such as non-replicating bacteria, fatty acids, triglycerides, lactowolfberry, antioxidants, vitamins, minerals, polyphenolics, flavonoids, EGCg, pycnogenol, α- and β-glucans, alpha-hydroxyisocaproate, aloe, honey, amino acids, branched chain amino acids or a combination thereof.

In an embodiment, the nutritional composition is in an administrable form such as pharmaceutical formulations, nutritional formulations, dietary supplements, functional foods and beverage products.

In another embodiment, the present disclosure provides a method of making a nutritional composition. The method comprises adding a high protein component and an exogenous nucleotide to a nutritional composition.

The nutrition composition can be administered to provide the exogenous nucleotide in an amount ranging from about 0.2 g/day to about 4 grams/day. The wound can be from pressure ulcers, surgical incisions, cuts, scrapes or a combination thereof. The mammal can also have diabetes.

An advantage of the present disclosure is to provide an improved nutritional composition having a high protein component and exogenous nucleotides.

Another advantage of the present disclosure is to provide a method of making an improved nutritional composition.

Yet another advantage of the present disclosure is to provide a nutritional composition that accelerates or improves wound healing in a mammal.

Additional features and advantages are described herein, and will be apparent from the following Detailed Description.

### DETAILED DESCRIPTION

The present disclosure relates to nutritional compositions including a high protein component and exogenous nucleotides and methods of making and using the nutritional compositions. Embodiments of the nutritional compositions of the present disclosure can provide improved healing of wounds. The wounds may be of any type including pressure ulcers, accident injury, surgical incisions, etc. The need to improve wound healing is evident from the high cost of wound care, longer hospital stays, and morbidity experienced by patients.

As used herein, the term "nutritional composition" includes, but is not limited to, complete nutritional compositions, partial or incomplete nutritional compositions, and disease or condition specific nutritional compositions.

As used herein, "about," is preferably understood to refer to numbers in a range of numerals. Moreover, all numerical ranges herein should be understood to include all integer, whole or fractions, within the range.

As used herein, "complete nutrition" are preferably nutritional products that contain sufficient types and levels of macronutrients (protein, fats and carbohydrates) and micronutrients to be sufficient to be a sole source of nutrition for the animal to which it is being administered to. Patients can receive 100% of their nutritional requirements from such complete nutritional compositions.

As used herein, "effective amount" is preferably an amount that prevents a deficiency, treats a disease or medical condition in an individual or, more generally, reduces symptoms, manages progression of the diseases or provides a nutritional, physiological, or medical benefit to the individual. A treatment can be patient- or doctor-related. In addition, while the terms "individual" and "patient" are often used herein to refer to a human, the invention is not so limited. Accordingly, the terms "individual" and "patient" refer to any animal, mammal or human having or at risk for a medical condition that can benefit from the treatment.

As used herein, "elderly" is preferably a human that is sixty-five years of age or older, more preferably seventy-five years or age or older.

As used herein, "incomplete nutrition" are preferably nutritional products that do not contain sufficient levels of macronutrients (protein, fats and carbohydrates) or micronutrients to be sufficient to be a sole source of nutrition for the animal to which it is being administered to. Partial or incomplete nutritional compositions can be used as a nutritional supplement.

As used herein, "Long term administrations" are preferably continuous administrations for more than 6 weeks.

As used herein, mammal, includes but is not limited to rodents, aquatic mammals, domestic animals such as dogs and cats, farm animals such as sheep, pigs, cows and horses, and humans. Wherein the term mammal is used, it is contemplated that it also applies to other animals that are capable of the effect exhibited or intended to be exhibited by the mammal.

As used herein the term "patient" is preferably understood to include an animal, especially a mammal, and more especially a human that is receiving or intended to receive treatment, as it is herein defined.

As used herein, "phytochemicals" or "phytonutrients" are non-nutritive compounds that are found in many foods. Phytochemicals are functional foods that have health benefits beyond basic nutrition, and are health promoting compounds that come from plant sources. As used herein, "Phytochemicals" and "Phytonutrients" refers to any chemical produced by a plant that imparts one or more health benefit on the user. Phytochemicals can be administered by any means, including topically, enterally, and/or parenterally. As used herein, non-limiting examples of phytochemicals and phytonutrients include those that are:
1. Phenolic compounds which include Monophenols (such as: Apiole, Carnosol, Carvacrol, Dillapiole, Rosemarinol); Flavonoids (polyphenols) including Flavonols (such as: Quercetin, Gingerol, Kaempferol, Myricetin, Rutin, Isorhamnetin), Flavanones (such as: Hesperidin, Naringenin, Silybin, Eriodictyol), Flavones (such as: Apigenin, Tangeritin, Luteolin), Flavan-3-ols (such as: Catechins, (+)-Catechin, (+)-Gallocatechin, (-)-Epicatechin, (-)-Epigallocatechin, (-)-Epigallocatechin gallate (EGCG), (-)-Epicatechin 3-gallate, Theaflavin, Theaflavin-3-gallate, Theaflavin-3'-gallate, Theaflavin-3,3'-digallate, Thearubigins), Anthocyanins (flavonals) and Anthocyanidins (such as: Pelargonidin, Peonidin, Cyanidin, Delphinidin, Malvidin, Petunidin), Isoflavones (phytoestrogens) (such as: Daidzein (formononetin), Genistein (biochanin A), Glycitein), Dihydroflavonols, Chalcones, Coumestans (phytoestrogens), and Coumestrol; Phenolic acids (such as: Ellagic acid, Gallic acid, Tannic acid, Vanillin, Curcumin); Hydroxycinnamic acids (such as: Caffeic acid, Chlorogenic acid, Cinnamic acid, Ferulic acid, Coumarin); Lignans (phytoestrogens), Silymarin, Secoisolariciresinol, Pinoresinol and lariciresinol); Tyrosol esters (such as: Tyrosol, Hydroxytyrosol, Oleocanthal, Oleuropein); Stilbenoids (such as: Resveratrol, Pterostilbene, Piceatannol) and Punicalagins;
2. Terpenes (isoprenoids) which include Carotenoids (tetraterpenoids) including Carotenes (such as: α-Carotene, β-Carotene, γ-Carotene, δ-Carotene, Lycopene, Neurosporene, Phytofluene, Phytoene), and Xanthophylls (such as: Canthaxanthin, Cryptoxanthin, Zeaxanthin, Astaxanthin, Lutein, Rubixanthin); Monoterpenes (such as: Limonene, Perillyl alcohol); Saponins; Lipids including : Phytosterols (such as: Campesterol, beta Sitosterol, gamma sitosterol, Stigmasterol), Tocopherols (vitamin E), and omega-3, 6, and 9 fatty acids (such as: gamma-linolenic acid); Triterpenoid (such as: Oleanolic acid, Ursolic acid, Betulinic acid, Moronic acid);
3. Betalains which include Betacyanins (such as: betanin, isobetanin, probetanin, neobetanin); and Betaxanthins (non glycosidic versions) (such as: Indicaxanthin, and Vulgaxanthin);
4. Organosulfides which include Dithiolthiones (isothiocyanates) (such as: Sulphoraphane); and Thiosulphonates (allium compounds) (such as: Allyl methyl trisulfide, and Diallyl sulfide), Indoles, glucosinolates which include Indole-3-carbinol; sulforaphane; 3,3'-Diindolylmethane; Sinigrin; Allicin; Alliin; Allyl isothiocyanate; Piperine; Syn-propanethial-S-oxide;
5. Protein inhibitors which include protease inhibitors;
6. Other organic acids which include Oxalic acid, Phytic acid (inositol hexaphosphate); Tartaric acid; and Anacardic acid; and
7. combinations thereof.

As used in this specification and the appended claims, the singular forms "a", "an" and "the" include plural referents unless the context clearly dictates otherwise. Thus, for example, reference to "a polypeptide" includes a mixture of two or more polypeptides, and the like.

As used herein, a "Prebiotic" is preferably a food substances that selectively promote the growth of beneficial bacteria or inhibit the growth of pathogenic bacteria in the intestines. They are not inactivated in the stomach and/or upper intestine or absorbed in the GI tract of the person ingesting them, but they are fermented by the gastrointestinal microflora and/or by probiotics. Prebiotics are for example defined by Glenn R. Gibson and Marcel B. Roberfroid, Dietary Modulation of the Human Colonic Microbiota: Introducing the Concept of Prebiotics, J. Nutr. 1995 125: 1401-1412. Examples of prebiotics include acacia gum, alpha glucan, arabinogalactans, beta glucan, dextrans, fructooligosaccharides, galactooligosaccharides, galactomannans, gentiooligosaccharides, glucooligosaccharides, guar gum, inulin, isomaltooligosaccharides, lactosucrose, lactulose, levan, maltodextrins, partially hydrolyzed guar gum, pecticoligosaccharides, retrograded starch, soyoligosaccharides, sugar alcohols, xylooligosaccharides, or a combination thereof.

As used herein, Probiotics micro-organisms (hereinafter "probiotics") are preferably microorganisms (alive, including semi-viable or weakened, and/or non-replicating), metabolites, microbial cell preparations or components of microbial cells that could confer health benefits on the host when administered in adequate amounts, more specifically, that beneficially affect a host by improving its intestinal microbial balance, leading to effects on the health or well-being of the host. (Salminen S, Ouwehand A. Benno Y. et al "Probiotics: how should they be defined" Trends Food Sci. Technol. 1999:10 107-10). In general, it is believed that these micro-organisms inhibit or influence the growth and/or metabolism of pathogenic bacteria in the intestinal tract. The probiotics may also activate the immune function of the host. For this reason, there have been many different approaches to include probiotics into food products. Examples of probiotics include Aerococcus, Aspergillus, Bacteroides, Bifidobacterium, Candida, Clostridium, Debaromyces, Enterococcus, Fusobacterium, Lactobacillus, Lactococcus, Leuconostoc, Melissococcus, Micrococcus, Mucor, Oenococcus, Pediococcus, Penicillium, Peptostrepococcus, Pichia, Propionibacterium, Pseudocatenulatum, Rhizopus, Saccharomyces, Staphylococcus, Streptococcus, Torulopsis, Weissella, or a combination thereof.

In a general embodiment, the present disclosure provides a nutritional composition comprising a high protein component and one or more exogenous nucleotides for use in treating a wound or improving wound healing in a mammal. The combination of a high protein content and one or more exogenous nucleotides can provide a synergistic benefit for accelerating or improving wound healing in a mammal. As used herein, the term "mammal" refers to a human (e.g., child, adult) or an animal.

Nucleotides are low molecular weight biological molecules key to biochemical processes. Sources include de novo synthesis, recovery via salvage mechanisms, and dietary intakes. While endogenous production serves as the main nucleotide source, there is evidence to suggest that exogenous sources are important in rapidly proliferating cells in the immune and gastrointestinal systems, where they may become conditionally essential. Exogenous nucleotides may support optimal growth and function of metabolically active cells in times of cellular insult and their supplementation may improve clinical outcomes in the critically ill and immune suppressed patient.

The exogenous nucleotides can be in the form of monomers and polymers as part of the nutritional compositions. A nucleotide is a subunit of deoxyribonucleic acid ("DNA") or ribonucleic acid ("RNA"). It is an organic compound made up of a nitrogenous base, a phosphate molecule, and a sugar molecule (deoxyribose in DNA and ribose in RNA). Individual nucleotide monomers (single units) are linked together to form polymers, or long chains. The exogenous nucleotides in embodiments of the present disclosure are specifically provided by dietary supplementation.

The exogenous nucleotides can be in a monomeric form such as, for example, 5' Adenosine Monophosphate ("5'-AMP"), 5'-Guanosine Monophosphate ("5'-GMP"), 5'-Cytosine Monophosphate ("5'-CMP"), 5'-Uracil Monophosphate ("5'-UMP"), 5'-Inosine Monophosphate ("5'-IMP"), 5'-Thymine Monophosphate ("5'-TMP") or a combination thereof. The exogenous nucleotides can also be in a polymeric form such as, for example, an intact RNA. There can be multiple sources of the polymeric form such as, for example, yeast RNA. In an embodiment, the exogenous nucleotide is in an amount of about 1 gram/1000 calories of the nutritional composition.

The high protein component includes one or more proteins, peptides, and amino acids, and precursors and metabolites of proteins, peptides, and amino acids in an amount that provides at least about 18% of the total calories of the nutritional composition.

The nutritional composition has a total volume of 300 mL or less. The high protein component in this nutritional composition includes at least one of proteins, peptides, and amino acids, and precursors and metabolites of proteins, peptides, and amino acids in an amount greater than about 14 grams.

In an embodiment, the high protein component includes at least about 5 grams of protein, at least about 3 grams of one or more bitter tasting amino acids, and at least about 7 grams of one or more neutral tasting acids. The bitter tasting amino acid can be arginine, phenylalanine, tyrosine, leucine, isoleucine, valine, methionine, histidine or a combination thereof. The neutral tasting acid can be glutamine, glycine, alanine, threonine, praline, serine or a combination thereof.

The term "protein", "peptide", "oligopeptides" or "polypeptide" as used herein is preferably understood to refer to any composition that includes, a single amino acids (monomers), two or more amino acids joined together by a peptide bond (dipeptide, tripeptide, or polypeptide), collagen, precursor, homolog, analog, mimetic, salt, prodrug, metabolite, or fragment thereof or combination. For the sake of clarity, the use of any of the above terms is interchangeable unless otherwise specified. It will be appreciated that polypeptides (or peptides or proteins or oligopeptides) often contain amino acids other than the 20 amino acids commonly referred to as the 20 naturally occurring amino acids, and that many amino acids, including the terminal amino acids, may be modified in a given polypeptide, either by natural processes such as glycosylation and other post-translational modifications, or by chemical modification techniques which are well known in the art. Among the known modifications which may be present in polypeptides of the present invention include, but are not limited to, acetylation, acylation, ADP-ribosylation, amidation, covalent attachment of a flavanoid or a heme moiety, covalent attachment of a polynucleotide or polynucleotide derivative, covalent attachment of a lipid or lipid derivative, covalent attachment of phosphatidylinositol, cross-linking, cyclization, disulfide bond formation, demethylation, formation of covalent cross-links, formation of cystine, formation of pyroglutamate, formylation, gamma-carboxylation, glycation, glycosylation, glycosylphosphatidyl inositol (GPI) membrane anchor formation, hydroxylation, iodination, methylation, myristoylation, oxidation, proteolytic processing, phosphorylation, prenylation, racemization, selenoylation, sulfation, transfer-RNA mediated addition of amino acids to polypeptides such as arginylation, and ubiquitination. The term "protein" also includes "artificial proteins" which refers to linear or non-linear polypeptides, consisting of alternating repeats of a peptide Non-limiting examples of proteins include dairy based proteins and plant based proteins. Dairy based proteins include casein, caseinates (e.g., all forms including sodium, calcium, potassium caseinates), whey (e.g., all forms including concentrate, isolate, demineralized), milk protein concentrate, and milk protein isolate. Plant based proteins include soy protein (e.g., all forms including concentrate and isolate), pea protein (e.g., all forms including concentrate and isolate), canola protein (e.g., all forms including concentrate and isolate), other plant proteins that commercially are wheat and fractionated wheat proteins, corn and it fractions including zein, rice, oat, potato, peanut, and any proteins derived from beans, lentils, and pulses.

Additional examples of proteins can come from animal sources (e.g., meats, meat powders, organ meats, and extracts of the meats), marine sources (e.g., fish meal, fish muscle, and the same for all forms of marine animals), and microbial sources (e.g., single cell proteins and variations).

All of the proteins listed above can be used as intact proteins or as hydrolysates. The hydrolysates can have a low to high degree of hydrolysis. The peptides that result from the hydrolysis can include large or small peptides or a range of peptide sizes.

Free amino acids can be used in place of or in addition to proteins to provide the necessary amino acids. Dipeptides are also commercially available and can provide the necessary amino acids.

Non-limiting examples of peptides can range in size categories including small (e.g., di and tripeptides that require no additional breakdown for absorption in the intestine), medium (e.g., oligopeptides that are 4 - 10 amino acids long) large (e.g., >10 amino acids but smaller than small proteins - a molecular weight range of 1,000 - 5,000), and very large (e.g., molecular weights greater than 5,000 but not intake protein).

Additional ingredients may be used with the exogenous nucleotides and the high protein component to improve wound healing. In an embodiment, the nutritional composition further comprises one or more ingredients such as non-replicating bacteria, fatty acids, triglycerides, lactowolfberry, antioxidants, vitamins, minerals, polyphenolics, flavonoids, EGCg, pycnogenol, α- and β-glucans, alpha-hydroxyisocaproate, aloe, honey, amino acids, branched chain amino acids or a combination thereof.

Specific ingredients and their functions are described in more detail as follows:
Nonreplicating bacteria (e.g., inactivated bacteria) - support of immune function.
Arginine - precursor of proline via ornithine, precursor of nitric oxide for vasodilation, and immune function via T-cell stimulation.
Glutamine - precursor of arginine via citrulline; lymphokine activated killer ("LAK") cell are dependent on glutamine for activity.
Fatty acids and triglycerides - modulation of inflammation and component of cell membranes and skin.
Lactowolfberry - Nestle® bioavailable form of goji berry for immune responsiveness.
Copper - essential for macrophage function and proliferation of immune cells in response to challenge.
Zinc - required for optimal innate immune response, essential co-factor for protein synthesis, cell division, and immune function.
Vitamin C - depleted by stress; needed for collagen synthesis, stimulation of interferon, maintenance of redox integrity of the cell and intracellular matrix components.
Vitamin A - important for regulation of antibody response as well as differentiation of B-cells to IgG-expressing cells, also needed for skin maintenance and collagen cross linking.
β-carotene - precursor of Vitamin A, benefits same as for vitamin A plus antioxidant activity.
Vitamin E - lipid soluble antioxidant stabilizes cell membranes and lipids to prevent oxidative damage to immune cells.
Selenium - component of selenoproteins, augments cellular immune response through production of interferon γ, earlier peak of T cell proliferation, and increased T helper cells.
Polyphenolics - inhibit inflammatory cytokine, interleukin-1 and decrease production of prostaglandin E2.
Flavonoids - stimulate or inhibit protein phosphorylation and thereby regulate immune cell function.
EGCg - catechin with protective effects on dendritic cells that survey wound site, reduces the production of inflammatory mediators IL-6 and COX-2.
Pycnogenol - improves both T-cell and B-cell function and stimulates IL-2 and Natural Killer T-cell activity.
α- and/or β-glucans - immune cell stimulators of both innate and acquired immune systems via binding macrophages directly.
AHCC - (methylated α-glucan) - specifically modified alpha-glucan.
Aloe - suppression of inflammatory cytokines TNF-alpha and IL-1 beta.
Honey - osmotic and pH effects to prevent infection and immune stimulation.
Branched chain amino acids - readily oxidized and therefore supplemented for the synthesis of proteins by immune cells, such as protein antibodies ("Ab") by B-cells.
Leucine - Most effective branched chain amino acid for the stimulation and maintenance of protein for immune cell proliferation and muscle anabolism.
α -HICA - (α-Hydroxyisocaproic acid) metabolite of leucine that can improve anabolism.

In an embodiment, the nutritional composition further includes one or more amino acids. Non-limiting examples of amino acids include Alanine, Arginine, Asparagine, Aspartate, Citrulline, Cysteine, Glutamate, Glutamine, Glycine, Histidine, Hydroxyproline, Hydroxyserine, Hydroxytyrosine, Hydroxylysine, Isoleucine, Leucine, Lysine, Methionine, Phenylalanine, Proline, Serine, Taurine, Threonine, Tryptophan, Tyrosine, and Valine and combination thereof.

As used herein the term "antioxidant" is preferably understood to include any one or more of various substances (as beta-carotene (a vitamin A precursor), vitamin C, vitamin E, and selenium) that inhibit oxidation or reactions promoted by Reactive Oxygen Species (ROS) and other radical and non-radical species. Additionally, antioxidants are molecules capable of slowing or preventing the oxidation of other molecules. As used herein, non-limiting examples of antioxidants include carotenoids, coenzyme Q10 ("CoQ10"), flavonoids, glutathione Goji (Wolfberry), hesperidine, Lactowolfberry, lignan, lutein, lycopene, polyphenols, selenium, vitamin A, vitamin B1, vitamin B6, vitamin B12, vitamin C, vitamin D, vitamin E, and combinations thereof

As used herein the term "vitamin" is preferably understood to include any of various fat-soluble or water-soluble organic substances (non-limiting examples include vitamin A , Vitamin B1 (thiamine), Vitamin B2 (riboflavin), Vitamin B3 (niacin or niacinamide), Vitamin B5 (pantothenic acid), Vitamin B6 (pyridoxine, pyridoxal, or pyridoxamine, or pyridoxine hydrochloride), Vitamin B7 (biotin), Vitamin B9 (folic acid), and Vitamin B12 (various cobalamins; commonly cyanocobalamin in vitamin supplements), vitamin C, vitamin D, vitamin E, vitamin K (including Vitamin K1 and Vitamin K2), essential in minute amounts for normal growth and activity of the body and obtained naturally from plant and animal foods or synthetically made, pro-vitamins, derivatives, analogs. As used herein the term "minerals" is preferably understood to include boron, calcium, chromium, copper, iodine, iron, magnesium, manganese, molybdenum, nickel, phosphorus, potassium, selenium, silicon, tin, vanadium, and zinc.

As used herein, the terms "treatment", "treat" and "to alleviate" is preferably to both prophylactic or preventive treatment (that prevent and/or slow the development of a targeted pathologic condition or disorder) and curative, therapeutic or disease-modifying treatment, including therapeutic measures that cure, slow down, lessen symptoms of, and/or halt progression of a diagnosed pathologic condition or disorder; and treatment of patients at risk of contracting a disease or suspected to have contracted a disease, as well as patients who are ill or have been diagnosed as suffering from a disease or medical condition. The term does not necessarily imply that a subject is treated until total recovery. The terms "treatment" and "treat" also refer to the maintenance and/or promotion of health in an individual not suffering from a disease but who may be susceptible to the development of an unhealthy condition, such as nitrogen imbalance or muscle loss. The terms "treatment", "treat" and "to alleviate" are also intended to include the potentiation or otherwise enhancement of one or more primary prophylactic or therapeutic measure. The terms "treatment", "treat" and "to alleviate" are further intended to include the dietary management of a disease or condition or the dietary management for prophylaxis or prevention a disease or condition

As used herein, a "tube feed" is preferably a complete or incomplete nutritional products that are administered to an animal's gastrointestinal system, other than through oral administration, including but not limited to a nasogastric tube, orogastric tube, gastric tube, jejunostomy tube (J-tube), percutaneous endoscopic gastrostomy (PEG), port, such as a chest wall port that provides access to the stomach, jejunum and other suitable access ports.

The nutritional composition can be in an administerable form such as pharmaceutical formulations, nutritional formulations, dietary supplements, functional foods, beverage products or a combination thereof.

The nutrition composition can be administered to provide the exogenous nucleotide in an amount ranging from about 0.2 g/day to about 4 grams/day. The wound can be from pressure ulcers, surgical incisions, cuts, scrapes or a combination thereof. The mammal can also have diabetes.

In an alternative embodiment, the present disclosure provides a method of improving pressure ulcers in a mammal using the same steps previously described.

Other optional ingredients can be added to make the nutritional composition sufficiently palatable. The optional ingredients can be added in any suitable amount.

### EXAMPLES

By way of example and not limitation, the following examples are illustrative of various embodiments of the present disclosure.

### EXAMPLE 1

**Table 1: Complete feeding product appropriate for dietary management of pressure ulcers**

| **Ingredient** | **Amount** | **Function** |
|---|---|---|
| Caseinate | 75 g (20% of energy) | High quality protein |
| Canola oil | 50 g (30% of energy) | Fatty acid source and energy |
| Maltodextrin | 188 g (50% of energy) | Carbohydrate and energy source |
| Yeast extract | 2.5 g | RNA/nucleotide source |
| Vitamin premix | 2.5 g | |
| Mineral premix | 1.0 g | |
| Emulsifier | 1.5 g | |
| Water | 1290 g | |
| Optional ingredients | | As listed elsewhere |

### EXAMPLE 2

**Table 2: Formulation for dietary management of pressure ulcers, one day of feeding**

| **Ingredient** | **Amount** | **Function** |
|---|---|---|
| Sodium caseinate | 55 g | High quality protein |
| Calcium caseinate | 17 g | High quality protein |
| Palm kernel oil | 20 g | Medium chain triglycerides |
| Fish oil | 16.7 g | n-3 fatty acids to reduce inflammation |
| Sunflower oil | 5.0 g | Fatty acids and energy |
| Maltodextrin | 200 g | Carbohydrate and energy source |
| Yeast extract | 2.26 g | RNA/nucleotide source |
| Arginine | 18.75 g | Support T-cell function |
| Vitamin premix | 2.5 g | |
| Mineral premix | 1.0 g | |
| Emulsifier | 1.5 g | |
| Water | 1243 g | |
| Optional ingredients | 52 g | As listed elsewhere |

### EXAMPLE 3

**Table 3: High protein oral nutritional supplement product, 8 fluid ounces**

| **Ingredient** | **Amount** | **Function** |
|---|---|---|
| Milk protein concentrate | 15 g | High quality protein |
| Oil blend | 6 g | Fatty acids and energy |
| Sucrose | 13 g | Carbohydrate and energy source |
| Corn syrup solids | 18.9 g | Carbohydrate and sweetness |
| Minerals | 2.5 g | |
| Yeast extract | 1.0 g | RNA/nucleotide source |
| Vitamins | 1.0 g | |
| Water | 200 g | |
| Optional ingredients | | As listed elsewhere |

### EXAMPLE 4

**Table 4: Protein and nucleotide modular oral nutrition product for wound healing**

| **Ingredient** | **Amount** | **Function** |
|---|---|---|
| Whey protein isolate | 6 g | High quality protein |
| Yeast extract | 2 g | RNA/nucleotide source |
| Citric acid | 1 g | Acidulent |
| Vitamin premix | 0.1 g | |
| Mineral premix | 0.2 g | |
| Optional ingredients | 0.4 g | As listed elsewhere |

### EXAMPLE 5

**Table 5: Amino acid and nucleotide modular oral nutrition product for wound healing**

| **Ingredient** | **Amount** | **Function** |
|---|---|---|
| L-arginine | 4 g | High quality protein |
| Yeast extract | 2 g | RNA/nucleotide source |
| Citric acid | 2 g | Acidulent |
| Malic acid | 1 g | Acidulent |
| Vitamin premix | 0.1 g | |
| Mineral premix | 0.2 g | |
| Optional ingredients | 0.4 g | As listed elsewhere |

Nutritional products is preferably understood to further include any number of optional additional ingredients, including conventional food additives, for example one or more, acidulants, additional thickeners, buffers or agents for pH adjustment, chelating agents, colorants, emulsifies, excipient, flavor agent, mineral, osmotic agents, a pharmaceutically acceptable carrier, preservatives, stabilizers, sugar, sweeteners, texturizers, and/or vitamin. The optional ingredients can be added in any suitable amount.

The nutritional composition according to the present invention comprises a high protein component and an exogenous nucleotide wherein the high protein component includes at least one of proteins, peptides, and amino acids, and precursors and metabolites of proteins, peptides, and amino acids in an amount that provides at least about 18% of the total calories of the nutritional composition, wherein the nutritional composition has a total volume of 300 mL or less and wherein the high protein component includes at least one of proteins, peptides, and amino acids, and precursors and metabolites of proteins, peptides, and amino acids in an amount greater than about 14 grams. In a further embodiment, the high protein component includes at least about 5 grams of protein, at least about 3 grams of a bitter tasting amino acid, and at least about 7 grams of a neutral tasting acid. In a further embodiment, the bitter tasting amino acid is selected from the group consisting of arginine, phenylalanine, tyrosine, leucine, isoleucine, valine, methionine, histidine and combinations thereof. In a further embodiment, the neutral tasting acid is selected from the group consisting of glutamine, glycine, alanine, threonine, praline, serine and combinations thereof. In a further embodiment, the exogenous nucleotide is in an amount of about 1 gram/1000 calories of the nutritional composition. In a further embodiment, the exogenous nucleotide is in a monomeric form selected from the group consisting of 5' Adenosine Monophosphate, 5'-Guanosine Monophosphate, 5'-Cytosine Monophosphate, 5'-Uracil Monophosphate, 5'-Inosine Monophosphate, 5'-Thymine Monophosphate and combinations thereof. In a further embodiment, the exogenous nucleotide is intact ribonucleic acid and/or other forms that contain nucleotides.

In a preferred embodiment, the nutritional composition further comprises an ingredient selected from the group consisting of non-replicating bacteria, fatty acids, triglycerides, lactowolfberry, antioxidants, vitamins, minerals, polyphenolics, flavonoids, EGCg, pycnogenol, α- and β-glucans, alpha-hydroxyisocaproate, aloe, honey, amino acids, branched chain amino acids and combinations thereof.

In a preferred embodiment, the nutritional composition is in an administerable form selected from the group consisting of pharmaceutical formulations, nutritional formulations, dietary supplements, functional foods and beverage products.

In a preferred embodiment, the nutritional composition further comprises antioxidants. In a further embodiment, the antioxidants are selected from a group consisting of: carotenoids, coenzyme Q10 ("CoQ10"), flavonoids, glutathione Goji (Wolfberry), hesperidine, Lactowolfberry, lignan, lutein, lycopene, polyphenols, selenium, vitamin A, vitamin B1, vitamin B6, vitamin B12, vitamin C, vitamin D, vitamin E, or combinations thereof.

In a preferred embodiment, the nutritional composition further comprises carbohydrate.

In a preferred embodiment, the nutritional composition further comprises fats.

In a preferred embodiment, the nutritional composition further comprises fish oils. In a further embodiment, the fish oils are selected from a group consisting of: docosahexaenoic acid ("DHA") and eicosapentaenoic acid ("EPA").

In a preferred embodiment, the nutritional composition further comprises minerals. In a further embodiment, the minerals are selected from a group consisting of: boron, calcium, chromium, copper, iodine, iron, magnesium, manganese, molybdenum, nickel, phosphorus, potassium, selenium, silicon, tin, vanadium, zinc, and combinations thereof.

In a preferred embodiment, the nutritional composition further comprises phytonutrients. In a further embodiment, the phytonutrients are selected from a group consisting of: flavonoids and allied phenolic and polyphenolic compounds, terpenoids including carotenoids, and alkaloids; including curcumin, limonin, and quercetin and combinations thereof.

In a preferred embodiment, the nutritional composition further comprises prebiotics. In a further embodiment, the prebiotics are selected from a group consisting of: acacia gum, alpha glucan, arabinogalactans, beta glucan, dextrans, fructooligosaccharides, galactooligosaccharides, galactomannans, gentiooligosaccharides, glucooligosaccharides, guar gum, inulin, isomaltooligosaccharides, lactosucrose, lactulose, levan, maltodextrins, partially hydrolyzed guar gum, pecticoligosaccharides, retrograded starch, soyoligosaccharides, sugar alcohols, xylooligosaccharides, or a combination thereof.

In a preferred embodiment, the nutritional composition further comprises probiotics. In a further embodiment, the probiotics are selected from a group consisting of, replications and non-replicating: Aerococcus, Aspergillus, Bacteroides, Bifidobacterium, Candida, Clostridium, Debaromyces, Enterococcus, Fusobacterium, Lactobacillus, Lactococcus, Leuconostoc, Melissococcus, Micrococcus, Mucor, Oenococcus, Pediococcus, Penicillium, Peptostrepococcus, Pichia, Propionibacterium, Pseudocatenulatum, Rhizopus, Saccharomyces, Staphylococcus, Streptococcus, Torulopsis, Weissella, or a combination thereof.

In a preferred embodiment, the nutritional composition further comprises vitamins. In a further embodiment, the vitamins are selected from a group consisting of: fat-soluble or water-soluble organic substances including vitamin A , vitamin B1, vitamin B2, vitamin B6, vitamin B12, vitamin C, vitamin D, vitamin E, vitamin K (including Vitamin K1 and Vitamin K2), folic acid and biotin, pro-vitamins, derivatives, analogs. And combinations thereof

In a preferred embodiment, the nutritional composition is complete nutrition.

In a preferred embodiment, the nutritional composition further comprises is incomplete nutrition.

The method of making a nutritional composition, comprises adding a high protein component and an exogenous nucleotide to a nutritional composition., The high protein component includes at least one of proteins, peptides, and amino acids, and precursors and metabolites of proteins, peptides, and amino acids in an amount that provides at least 18% of the total calories of the nutritional composition and the nutritional composition has a total volume of 300 mL or less and wherein the high protein component includes at least one of proteins, peptides, and amino acids, and precursors and metabolites of proteins, peptides, and amino acids in an amount greater than about 14 grams.

In a further embodiment, the high protein component includes at least about 5 grams of protein, at least about 3 grams of a bitter tasting amino acid, and at least about 7 grams of a neutral tasting acid. In a further embodiment, the nutritional composition is in an administerable form selected from the group consisting of pharmaceutical formulations, nutritional formulations, dietary supplements, functional foods and beverage products.

In a further embodiment, the nutrition composition is administered to provide the exogenous nucleotide in an amount ranging from about 0.2 g/day to about 4 grams/day. In a further embodiment, the mammal is a human. In a further embodiment, the mammal is elderly. In a further embodiment, the mammal is in a hospital. In a further embodiment, the mammal is in a care facility. In a further embodiment, the mammal is in a nursing home. In a further embodiment, the nutritional composition is a tube feed. In a further embodiment, the administering is for long-term administration. In a further embodiment, the administering is for short-term administration..

In a further embodiment, the wound is a pressure ulcers, surgical incisions, cuts, scrapes and combinations thereof.

In a further embodiment, the mammal has diabetes. In a further embodiment, the mammal has renal failure. In a further embodiment, the mammal has hepatic insufficiency or hepatic failure.

In a preferred embodiment, is wherein the method leads to a decrease in healthcare spending costs. In a further embodiment, the decrease in healthcare spending costs is due to decreased length of stay in a hospital. In a further embodiment, the decrease in healthcare spending costs is due to decreased length of stay in a care facility. In a further embodiment, the decrease in healthcare spending costs is due to decreased complications.

All dosage ranges contained within this application are intended to include all numbers, whole or fractions, contained within said range.

It should be understood that various changes and modifications to the presently preferred embodiments described herein will be apparent to those skilled in the art.

## Claims

1. A nutritional composition comprising a high protein component and an exogenous nucleotide wherein the high protein component includes at least one of proteins, peptides, and amino acids, and precursors and metabolites of proteins, peptides, and amino acids in an amount that provides at least about 18% of the total calories of the nutritional composition, wherein the nutritional composition has a total volume of 300 mL or less and wherein the high protein component is in an amount greater than about 14 grams, for use in treating a wound or improving wound healing in a mammal.

2. The nutritional composition of Claim 1, wherein the high protein component includes at least about 5 grams of protein, at least about 3 grams of a bitter tasting amino acid, and at least about 7 grams of a neutral tasting acid;
wherein the bitter tasting amino acid is selected from the group consisting of arginine, phenylalanine, tyrosine, leucine, isoleucine, valine, methionine, histidine and combinations thereof; and
herein the neutral tasting acid is selected from the group consisting of glutamine, glycine, alanine, threonine, proline, serine and combinations thereof.

3. The nutritional composition of Claim 1, wherein the exogenous nucleotide is in an amount of about 1 gram/1000 calories of the nutritional composition.

4. The nutritional composition of Claim 1, wherein the exogenous nucleotide is in a monomeric form selected from the group consisting of 5' Adenosine Monophosphate, 5'-Guanosine Monophosphate, 5'-Cytosine Monophosphate, 5'-Uracil Monophosphate, 5'-Inosine Monophosphate, 5'-Thymine Monophosphate and combinations thereof.

5. The nutritional composition of Claim 1, wherein the exogenous nucleotide is intact ribonucleic acid and/or other forms that contain nucleotides.

6. The nutritional composition of Claim 1 further comprising an ingredient selected from the group consisting of non-replicating bacteria, probiotics, fatty acids, triglycerides, Goji, lactowolfberry, antioxidants, vitamins, minerals, polyphenolics, flavonoids, EGCg, pycnogenol, α- and β-glucans, alpha-hydroxyisocaproate, aloe, honey, amino acids, branched chain amino acids, carbohydrates, fats, fish oils, phytonutrients, prebiotics, and combinations thereof.

7. The nutritional composition according to any one of claims 1 to 6, wherein the nutrition composition provides the exogenous nucleotide in an amount ranging from about 0.2 g/day to about 4 grams/day.

8. The nutritional composition according to any one of claims 1 to 6 for use in treating a wound or improving wound healing in a mammal wherein said mammal is elderly.

9. The composition according to any one of claims 1 to 6, for use in treating a wound or improving wound healing in a mammal wherein said mammal is in a hospital, care facility, nursing home, or undergoing in-home care.

10. The nutritional composition according to any one of claims 1 to 6, wherein said nutritional composition is a tube feed.

11. The nutritional composition according to any one of claims 1 to 6, for use in treating a wound or improving wound healing in a mammal, wherein the mammal has at least one of diabetes, renal failure, hepatic insufficiency, or hepatic failure.

12. The nutritional composition according to any one of claims 1 to 6, for use in treating a wound or improving wound healing in a mammal, wherein the wound is a pressure ulcers or a surgical incision.

## Patentansprüche

1. Nahrungszusammensetzung, die eine proteinreiche Komponente und ein exogenes Nukleotid umfasst, wobei die proteinreiche Komponente mindestens eines von Proteinen, Peptiden und Aminosäuren und Ausgangsstoffen und Stoffwechselprodukten von Proteinen, Peptiden und Aminosäuren in einer Menge enthält, die mindestens 18 % der Gesamtkalorien der Nahrungszusammensetzung bereitstellt, wobei die Nahrungszusammensetzung ein Gesamtvolumen von 300 ml oder weniger aufweist und wobei die proteinreiche Komponente in einer Menge größer als ungefähr 14 Gramm vorliegt, für die Nutzung zur Wundbehandlung oder Verbesserung der Wundheilung bei einem Säuger.

2. Nahrungszusammensetzung nach Anspruch 1, wobei die proteinreiche Komponente mindestens ungefähr 5 Gramm Protein, mindestens ungefähr 3 Gramm einer bitter schmeckenden Aminosäure und mindestens ungefähr 7 Gramm einer neutral schmeckenden Säure enthält;
wobei die bitter schmeckende Aminosäure aus der Gruppe ausgewählt ist, die aus Arginin, Phenylalanin, Tyrosin, Leucin, Isoleucin, Valin, Methionin, Histidin und Kombinationen daraus besteht; und
worin die neutral schmeckende Säure aus der Gruppe ausgewählt ist, die aus Glutamin, Glycin, Alanin, Threonin, Prolin, Serin und Kombinationen daraus besteht.

3. Nahrungszusammensetzung nach Anspruch 1, wobei das exogene Nukleotid in einer Menge von ungefähr 1 Gramm/1000 Kalorien der Nahrungszusammensetzung vorliegt.

4. Nahrungszusammensetzung nach Anspruch 1, wobei das exogene Nukleotid in einer monomeren Form vorliegt, ausgewählt aus der Gruppe, die aus 5'-Adenosin-Monophosphat, 5'-Guanosin-Monophosphat, 5'-Cytosin-Monophosphat, 5'-Uracil-Monophosphat, 5'-Inosin-Monophosphat, 5'-Thymin-Monophosphat und Kombinationen daraus besteht.

5. Nahrungszusammensetzung nach Anspruch 1, wobei das exogene Nukleotid eine intakte Ribonukleinsäure und/oder andere Formen, die Nukleotide enthalten, ist.

6. Nahrungszusammensetzung nach Anspruch 1, die des Weiteren einen Inhaltsstoff aus der Gruppe enthält, die aus nicht replizierenden Bakterien, Probiotika, Fettsäuren, Triglyceriden, Goji, Lactowolfberry, Antioxidantien, Vitaminen, Mineralien, Polyphenolen, Flavonoiden, EGCg, Pycnogenol, α- und β-Glukanen, Alpha-Hydroxyisocaproat, Aloe, Honig, Aminosäuren, verzweigten Aminosäurenketten, Kohlenhydraten, Fetten, Fischölen, sekundären Pflanzeninhaltsstoffen, Präbiotika und Kombinationen daraus besteht.

7. Nahrungszusammensetzung nach einem der Ansprüche 1 bis 6, wobei die Nahrungszusammensetzung die exogenen Nukleotide in einer Menge bereitstellt, die zwischen ungefähr 0,2 g/Tag bis ungefähr 4 g/Tag liegt.

8. Nahrungszusammensetzung nach einem der Ansprüche 1 bis 6 zur Nutzung in der Wundbehandlung oder der Verbesserung der Wundheilung bei einem Säuger, wobei der Säuger älter ist.

9. Zusammensetzung nach einem der Ansprüche 1 bis 6 zur Nutzung in der Wundbehandlung oder der Verbesserung der Wundheilung bei einem Säuger, wobei sich der Säuger in einem Krankenhaus, einer Pflegeeinrichtung, einem Pflegeheim befindet oder unter häuslicher Pflege steht.

10. Nahrungszusammensetzung nach einem der Ansprüche 1 bis 6, wobei die Nahrungszusammensetzung eine Sondennahrung ist.

11. Nahrungszusammensetzung nach einem der Ansprüche 1 bis 6 zur Nutzung in der Wundbehandlung oder der Verbesserung der Wundheilung bei einem Säuger, wobei der Säuger mindestens eines unter Diabetes, Nierenversagen, Leberinsuffizienz oder Leberversagen aufweist.

12. Nahrungszusammensetzung nach einem der Ansprüche 1 bis 6 zur Nutzung in der Wundbehandlung oder der Verbesserung der Wundheilung bei einem Säuger, wobei die Wunde ein Druckulcus oder eine chirurgische Inzision ist.

## Revendications

1. Composition nutritionnelle comprenant un composant à haute teneur en protéines et un nucléotide exogène où le composant à haute teneur en protéines inclut au moins un parmi des protéines, peptides et acides aminés, et précurseurs et métabolites de protéines, peptides et acides aminés en une quantité qui fournit au moins environ 18 % des calories totales de la composition nutritionnelle, où la composition nutritionnelle a un volume total de 300 ml ou moins et où le composant à haute teneur en protéines est en une quantité supérieure à environ 14 grammes, destinée à être utilisée pour le traitement d'une blessure ou l'amélioration de la cicatrisation de plaies chez un mammifère.

2. Composition nutritionnelle selon la revendication 1, où le composant à haute teneur en protéines inclut au moins environ 5 grammes de protéine, au moins environ 3 grammes d'un acide aminé au goût amer, et au moins environ 7 grammes d'un acide au goût neutre ;
dans laquelle l'acide aminé au goût amer est choisi dans le groupe constitué d'arginine, phénylalanine, tyrosine, leucine, isoleucine, valine, méthionine, histidine et leurs combinaisons ; et
dans laquelle l'acide au goût neutre est choisi dans le groupe constitué de glutamine, glycine, alanine, thréonine, proline, sérine et leurs combinaisons.

3. Composition nutritionnelle selon la revendication 1, où le nucléotide exogène est en une quantité d'environ 1 gramme/1000 calories de la composition nutritionnelle.

4. Composition nutritionnelle selon la revendication 1, où le nucléotide exogène est sous une forme monomère choisie dans le groupe constitué de 5' adénosine monophosphate, 5'-guanosine monophosphate, 5'-cytosine monophosphate, 5'-uracile monophosphate, 5'-inosine monophosphate, 5'-thymine monophosphate et leurs combinaisons.

5. Composition nutritionnelle selon la revendication 1, où le nucléotide exogène est un acide ribonucléique intact et/ou d'autres formes qui contiennent des nucléotides.

6. Composition nutritionnelle selon la revendication 1, comprenant en outre un ingrédient choisi dans le groupe constitué de bactéries ne se répliquant pas, probiotiques, acides gras, triglycérides, goji, lacto-symphorine occidentale, antioxydants, vitamines, minéraux, composés polyphénoliques, flavonoïdes, EGCg, pycnogénol, α- et β-glucanes, alpha-hydroxyisocaproate, aloès, miel, acides aminés, acides aminés à chaîne ramifiée, hydrates de carbone, graisses, huiles de poisson, phytonutriments, prébiotiques et leurs combinaisons.

7. Composition nutritionnelle selon l'une quelconque des revendications 1 à 6, où la composition de nutrition fournit le nucléotide exogène en une quantité allant d'environ 0,2 g/jour à environ 4 grammes/jour.

8. Composition nutritionnelle selon l'une quelconque des revendications 1 à 6, destinée à être utilisée pour le traitement d'une blessure ou l'amélioration de la cicatrisation de plaies chez un mammifère, ledit mammifère étant âgé.

9. Composition selon l'une quelconque des revendications 1 à 6, destinée à être utilisée pour le traitement d'une blessure ou l'amélioration de la cicatrisation de plaies chez un mammifère, ledit mammifère étant dans un hôpital, un établissement de soins, une maison de retraite, ou recevant des soins à domicile.

10. Composition nutritionnelle selon l'une quelconque des revendications 1 à 6, où ladite composition nutritionnelle est une sonde d'alimentation.

11. Composition nutritionnelle selon l'une quelconque des revendications 1 à 6, destinée à être utilisée pour le traitement d'une blessure ou l'amélioration de la cicatrisation de plaies chez un mammifère, où le mammifère présente au moins une affection parmi le diabète, une insuffisance rénale, une insuffisance hépatique ou une défaillance hépatique.

12. Composition nutritionnelle selon l'une quelconque des revendications 1 à 6, destinée à être utilisée pour le traitement d'une blessure ou l'amélioration de la cicatrisation de plaies chez un mammifère, où la blessure consiste en des ulcères de pression ou une incision chirurgicale.
